# EUROPEAN PATENT APPLICATION

(11) **EP 1 347 064 A2**
(43) Date of publication of application: **24.09.2003**
(21) Application number: 03006189.9
(22) Date of filing: 19.03.2003
(51) Int. Cl.: C12Q 1/70

(54) **Base sequence characteristic of small round-structured viruses**

(30) Priority: 20.03.2002 JP 2002078971
(71) Applicant: Tosoh Corporation, Shinnanyo-shi, Yamaguchi-ken, 746-8501 (JP)
(72) Inventor: Yasukawa, Kiyoshi, Kawasaki-shi, Kanagawa-ken (JP); Ishiguro, Takahiko, Yokohama-shi, Kanagawa-ken (JP)
(74) Representative: VOSSIUS & PARTNER

(57) **Abstract**

A cDNA sequence of any one of SEQ ID NOS:1 to 4 or a derivative thereof, which binds to a genogroup I small round-structured virus genome, or a fragment of a cDNA sequence of any one of SEQ ID NOS:1 to 4 or a derivative thereof, which is large enough to bind to a genogroup I small round-structured virus genome.

## Description

Small round structured viruses (SRSVs) are generally known as causative agents of viral food poisoning. The present invention relates to base sequences of SRSVs useful in clinical tests, hygienic tests, food tests and food poisoning tests.

Small round-structured viruses belong to the human Caliciviridae, which is divided into three genetic groups, genogroup I (GI), genogroup II (GII) and genogroup III (GIII). In general, GI and GII viruses are called SRSVs, while GIII viruses are called human caliciviruses (in a narrow sense).

Viruses are responsible for an estimated 20% of food poisoning cases reported in Japan. SRSVs are detected in over 80% of viral food poisoning cases. The leading infection source is food, and raw oysters are often problematic. Detection of SRSVs from infants and young children with (sporadic) acute gastroenteritis indicates transmissibility from human to human. Therefore, tests for SRSVs are an important issue in view of public hygiene and quality control of food.

To date, detection of SRSVs has been based on electron microscopy. However, this technique requires at least 10⁶ virus particles/ml to be present for successful detection and is limited to fecal specimens from patients. Besides, it is useful only for virus detection, but not for virus identification. The recent success in production of human calicivirus virus-like particles has triggered research for ELISA using them for detection of human calicivirus-specific antibodies in serum. However, it is no more sensitive than electron microscopy. Thus, conventional techniques are operationally complicated and time-consuming and can not achieve quick detection of traces of SRSVs in specimens.

Under the circumstances described above, the present inventors have proposed oligonucleotides that bind to intramolecularly free regions in the genomic RNAs of GI and GII SRSVs at relatively low and constant temperatures (35-50°C, preferably 41°C) and oligonucleotide primers and oligonucleotide probes for amplification-based detection of SRSV RNA. Further, the present inventors have proposed a method of detecting GI and GII SRSVs using isothermal RNA amplification (Japanese Patent Application JP2000-182326, Japanese Patent Application JP2000-359482 and Japanese Patent Application JP2001-020231). However, GI and GII SRSVs are further divided into various subtypes. Furthermore, the variety of base sequences are rich between strains both for GI and GII SRSVs. Base sequences of many strains are needed to evaluate the above-mentioned detection techniques and develop new techniques for detecting GI or GII SRSV with high coverage, but base sequences that have been known to date is limited.

To solve this problem, the present inventors have identified the base sequences of regions containing the regions amplified for detection of four GI SRSVs and ten GII SRSVs by the above-mentioned method by trial and error PCR using oligonucleotides. Namely, the present invention provides base sequences characteristic of small round-structured viruses. In order to attain the above-mentioned object, the present invention defined in Claim 1 of the present application provides a cDNA sequence of any one of SEQ ID NOS:1 to 4 or a derivative thereof, which binds to a GI SRSV genome, or a fragment of a cDNA sequence of any one of SEQ ID NOS:1 to 4 or a derivative thereof, which is large enough to bind to a genogroup I small round-structured virus genome. The present invention defined in Claim 2 of the present application provides a cDNA sequence of any one of SEQ ID NOS:5 to 14 or a derivative thereof, which binds to a GII SRSV genome, or a fragment of a cDNA sequence of any one of SEQ ID NOS:5 to 14 or a derivative thereof, which is large enough to bind to a genogroup II small round-structured virus genome. Now, the present invention will be described in detail.

An oligonucleotide selected based on the base sequences of the present invention may be used in any gene amplification methods without any particular restrictions, including DNA amplification methods such as PCR and RNA amplification methods such as NASBA and 3SR.

The base sequence of the above-mentioned oligonucleotide is not particularly limited. Screening for such gene sequences is exemplified in Japanese Patent Application JP2000-182326, Japanese Patent Application JP2000-359482 and Japanese Patent Application JP2001-020231, which disclose screening for oligonucleotide sequences hybridizable specifically to intramolecularly free regions in a target RNA.

There is no particular restriction on how to use the oligonucleotide thus obtained for gene amplification. It may be used, for example, as a scissor probe for RNA cleavage, an oligonucleotide primer for nucleic acid amplification, or as a probe after partial modification or attachment of a detectable label. Its use for isothermal RNA amplification is described specifically in Japanese Patent Application JP2000-182326 and Japanese Patent Application JP2000-359482.

Now, the present invention will be described in further detail by referring to Examples. However, the present invention is by no means restricted to these specific Examples.

### EXAMPLE 1 BASE SEQUENCING

RNAs were extracted from four fecal specimens from four GI SRSV-infected patients and ten fecal specimens from ten GII SRSV-infected patients by an ordinary method. For the GI SRSVs, to select the base sequences of SEQ ID NOS:1 and 2, PCR amplification using an oligonucleotide of SEQ ID NO:15 and an oligonucleotide of SEQ ID NO:16 was followed by sequencing of the resulting DNAs. To select the base sequences of the base sequences of SEQ ID NOS:1 and 2, PCR amplification using an oligonucleotide of SEQ ID NO:17 and an oligonucleotide of SEQ ID NO:18 was followed by sequencing of the resulting DNAS.

For the GII SRSVs, to select the base sequences of SEQ ID NOS:5 to 10, PCR amplification using an oligonucleotide of SEQ ID NO:19 and an oligonucleotide of SEQ ID NO:20 was followed by sequencing of the resulting DNAs. To select the base sequences of SEQ ID NOS:11 to 14, PCR amplification using an oligonucleotide of SEQ ID NO:21 and an oligonucleotide of SEQ ID NO:19 was followed by sequencing of the resulting DNAs.

As explained above, the base sequences of the present invention make it possible to develop oligonucleotides hybridizable to RNAs of genes of various GI SRSV strains and GII SRSV strains, which are useful for amplification and detection of SRSV genes.

The fragments of cDNA sequences of the present invention may be oligonucleotides consisting of at least 10 bases in the base sequences in the Sequence Listing. It is obvious from the fact base sequences of about 10 bases are sufficient to ensure for primers or probes the specificity for a target nucleic acid.

The entire disclosure of Japanese Patent Application No. 2002-78971 filed on March 20, 2002 including specification, claims and summary is incorporated herein by reference in its entirety.

## Claims

1. A cDNA sequence of any one of SEQ ID NOS:1 to 4 or a derivative thereof, which binds to a genogroup I small round-structured virus genome, or a fragment of a cDNA sequence of any one of SEQ ID NOS:1 to 4 or a derivative thereof, which is large enough to bind to a genogroup I small round-structured virus genome.

2. A cDNA sequence of any one of SEQ ID NOS:5 to 14 or a derivative thereof, which binds to a genogroup II small round-structured virus genome, or a fragment of a cDNA sequence of any one of SEQ ID NOS:5 to 14 or a derivative thereof, which is large enough to bind to a genogroup II small round-structured virus genome.


SEQUENCE LISTING

<110> Tosoh Corporation

<120> BASE SEQUENCE CHARACTERISTIC OF SMALL ROUND-STRUCTURED

<130> H 1502 EP

<160> 17

<210> 1
<211> 658
<212> DNA
<213> Human calicivirus

<220> DNA
<223>

<400> 1
ccaatgcaat tgaacaggtc atggacacac cagtggcatg gagctacagt gatgcttgca 60
tgtccctaga taagacaacc agttccggcc acccccatca taagaagaaa aatgatgact 120
ggaatggaaa ttcatttgtg agggaattgg gcgaccaggc ggcgcatgct aacagcatgt 180
acgaacttgg taagtccatg aaacctgtgt atacagcagc tctaaaggat gagctagtca 240
agccagataa ggtgtacaca aaaatcaaaa agagactgct ttggggcgcg gaccttggca 300
ccgtcattcg cgctgccaga gcatttgggc cgttctgtga ggccataaaa ccccatgtaa 360
tcaaattgcc tatcaaggtg ggtatgaatg ccatagagga tggtcctttg atttatgcag 420

agcactccaa atataagttt cattatgatg ctgactatac agcttgggac tcaacacaaa 480
acagggagat catgatggaa tccttcaaca ttatgtgtaa gctcacagcc aacccctcct 540
tggccgcagt ggtggcacag gatctactct ccccatctga aatggacgtt ggcgactatg 600
tgatcagtgt caaagatggt ctgccatctg gctttccatg cacttcacag gtgaatag 658

<210> 2
<211> 658
<212> DNA
<213> Human calicivirus

<220> DNA
<223>

<400> 2
ccaatgcaat tgaacaggtc atggacacac cagtggcatg gagctacagt gatgcttgca 60
tgtccctaga taagacaacc agttccggcc acccccatca taagaagaaa aatgatgact 120
ggaatggaaa ttcatttgtg agggaattgg gcgaccaggc ggcgcatgct aacagcatgt 180
acgaacttgg taagtccatg aaacctgtgt atacagcagc tctaaaggat gagctagtca 240
agccagataa ggtgtacaca aaaatcaaaa agagactgct ttggggcgcg gaccttggca 300
ccgtcattcg cgctgccaga gcatttgggc cgttctgtga ggccataaaa ccccatgtaa 360
tcaaattgcc tatcaaggtg ggtatgaatg ccatagagga tggtcctttg atttatgcag 420
agcactccaa atataagttt cattatgatg ctgactatac agcttgggac tcaacacaaa 480
acagggagat catgatggaa tccttcaaca ttatgtgtaa gctcacagcc aacccctcct 540
tggccgcagt ggtggcacag gatctactct ccccatctga aatggacgtt ggcgactatg 600
tgatcagtgt caaagatggt ctgccatctg gctttccatg cacttcacag gtgaatag 658

<210> 3

<211> 865

<212> DNA

<213> Human calicivirus

<220> DNA

<223>

<400> 3

gtattgaacc cgcataccta ggtggtaagg accctcgagt ccaaaatggt ccctccctcc 60

aacaggtgct ccgcgatcag ctgaaaccat tcgcagatcc tcgtgggcgc atgcctgagc 120

ctggcttact ggaggctgct gtagaaactg tgacatctat gttagaacag acaatggaca 180

ccccaagtcc gtggtcttat gctgatgcct gtcaatctct tgacaagacg actagttcag 240

gctatcccca ccataaaaga aagaatgatg attggaatgg cactaccttt gtcagagaac 300

tcggtgacca agcagcgcat gccaatagca tgtatgagaa tgccaaacac atgaagccca 360

tatacactgc agccctgaag gatgaattgg ttaagcctga gaaggtctac cagaaagtta 420

agaaacgcct gttgtggggc gccgatcttg ggacagtgat cagagctgcc agggcttttg 480

gcccattctg tgatgctata aagccacatg tcatcaaatt gccaataaaa gttggcatga 540

acacaataga ggatggccct ttaatttatg ctgaacatgc caagtacaaa aatcattttg 600

atgcagatta cacagcatgg gactctacac aaaatagaca aattatgaca gaatcctttt 660

ccatcatgtc acgcctcacg gcctctccag aactagctga ggttgtagcc caggacttac 720

tagcaccatc cgagatggat gtgggtgact atgttataag ggtcaaagaa ggcctaccat 780

caggatttcc ctgcacttct caagtgaata gcataaatca ctggataatc accctttgtg 840

cattgtctga ggctactggc tcatc 865

<210> 4

<211> 1113

<212> DNA

<213> Human calicivirus

<220> DNA

<223>

<400> 4

aatatccccg aaccactgcc ccctggggtc tatgaacccg cctacctcgg gggcccggga 60

ccctagggtg actggcggtc cctcactcca acaagtgttg cgggatcagt taaagccatt 120

tgctgagcca cgaggacgca tgccagagcc aggtctcttg gaggccgcag ttgagactgt 180

gacttcatca ttagagcagg ttatggacac tcccgttcct tggagctata gtgatgcgtg 240

ccagtccctt gataagacca ctagttctgg tttcccctac cacagaagga agaatgacga 300

ctggaatggc accacctttg ttagggagtt aggggagcag gcggcacacg ctaacaacat 360

gtatgagcag gctaagagta tgaaacccat gtacacggca gcacttaaag atgaactagt 420

caaaccagag aaggtatacc agaaagtgaa aaagcgcttg ttatgggggg cagacttggg 480

cacggtagtt cgggccgcac gggcttttgg cccattctgt gatgctataa aatcccacac 540

aatcaaattg cctatcaaag ttggaatgaa ttcaattgag gatgggccac tgatctatgc 600

agaacattca aaatataagt accactttga tgcagattac acagcttggg attcaactca 660

aaataggcaa atcatgacag agtcattttc aatcatgtgt cggctaactg catcacctga 720

actagcttca gtggtggctc aagacttgct cgcaccctca gagatggatg ttggcgacta 780

cgtcataaga gtgaaggaag gcctcccatc tggtttccca tgcacatcac aagttaatag 840

tataaaccat tggttaataa ctctgtgtgc cctttctgaa gtaactggtc tgtcgccaga 900

tgttatccag tccatgtcat atttctcttt ctatggtgat gatgaaatag tgtcaactga 960

catagaattt gatccagcaa aactgacaca agttctcaga gagtatggac ttaaacccac 1020

ccgccccgac aaaagcgagg gcccaataat tgtaagggaa gagtgtggat gggttggtct 1080

ttttacgtcg cactatctcc cgcgacgccg cgg 1113

<210> 5

<211> 620

<212> DNA

<213> Human calicivirus

<220> DNA

<223>

<400> 5

agttgtcgcg ggtgtccaca ccgcagcagc ccgggggggc aacactgtca tatgtgccac 60

tcaagggcaa gatggagagg cagtccttga gggtaatgag gacctcggca catactgcgg 120

tgctccaatc ctaggccctg gcaaggcgcc taaactcagc acgaagacca aattctggcg 180

ttcatcacca gatgccttgc cacctggcac atatgaacct gcttacctgg gaggcaagga 240

ccctagagtg gagaaaggac cctccctgca acaagttatg agagatcagc taaagccctt 300

cacagagcct aggggcaaac cacctagacc tgcagtctta gaagaagcta aaaagacagt 360

gatgaatgtc ctagaacaaa ccattgaccc tgctaagcca tggtcctact cgcaagcatg 420

tgcctcactg gacaaaacca cttctagtgg tagcccccac catgtcaaga aaaatgacca 480

ttggaatggg gagtccttca ctggccccct tgcagatcaa gcatctaaag ccaacctcat 540

gtatgaacag gccaagcatg tgcagcccgt gtacacggcc gcgctcaaag atgagcttgt 600

taagactgac aaaatttttt 620

<210> 6

<211> 620

<212> DNA

<213> Human calicivirus

<220> DNA

<223>

<400> 6

tgtggttatt ggagtccaca cggccgccgc tcgtggggga aacactgtca tatgtgccac 60

ccaggggagt gagggggagg ctacacttga aggtggtgac agtaagggaa catactgtgg 120

tgcaccaatc ctaggcccag gtagtgcccc aaaactcagc accaagacca aattctggag 180

atcgtccaca acaccactcc cacctggcac ctatgaacca gcctatctcg gtggtaagga 240

tcccagagtc aagggtggcc cttcattgca acaagtcatg agggatcagt tgaaaccatt 300

tacagagccc aggggcaaac caccaaagcc aagtgtgttg gaagctgcca agaaaaccat 360

catcaatgtc cttgaacaaa caatcgatcc acccgagaaa tggtcattca cgcaagcttg 420

cgcgtctctt gacaagacca cttccagtgg ccatccgcac cacatgcgga aaaacgactg 480

ctggaacggg gagtccttca caggcaagct ggcagaccag gcttccaagg ccaacctgat 540

gttcgaagag gggaagaaca tgaccccagt ctacacaggt gcgcttaagg atgagttagt 600

taagactgac aaaatttttt 620

<210> 7

<211> 616

<212> DNA

<213> Human calicivirus

<220> DNA

<223>

<400> 7

tgtggttatt ggagtcccac ggctgccgct cgtgggggga acactgtcat atgtgccacc 60

caggggagtg agggggaggc tacacttgaa ggtggtgaca gtaagggaac atactgtggt 120

gcaccaatcc taggcccagg gagtgcccca aaactcagca ccaagaccaa attctggaga 180

tcgtccacaa caccactccc acctggcacc tatgaaccag cctatcttgg tggtaaggac 240

cccagagtca agggtggccc ttcattgcaa caagttatga gggatcagtt gaaaccattt 300
acagagccca ggggcaaacc accaaagcca agtgtgttgg aagctgccaa gaaaaccatc 360
atcaatgtcc ttgaacaaac aatcgatcca cccgagaaat ggtcattcac gcaagcttgc 420
gcgtcccttg acaagaccac ttccagtggc catccacacc acatgcggaa aaacgactgc 480
tggaacgggg agtccttcac aggcaagctg gcagaccagg cttccaaggc caacctgatg 540
ttcgaagagg ggaagaacat gaccccagtc tacacaggtg cgcttaaggg atgagttagt 600
taagactgac aaaatt 616

<210> 8
<211> 617
<212> DNA
<213> Human calicivirus

<220> DNA
<223>

<400> 8
ggggatgact acgtggtcat tggggtccac acggctgctg cccgtggggg aaacactgtc 60
atatgtgcca cccagggaag tgaaggtgag gccactcttg agggcggcga tgacaaaggc 120
acttactgtg gtgccccaat cctaggtcca gggagtgctc caaagcttag caccaaaact 180
aagttttgga ggtcatccac aacaccactc ccacccggta cctatgaacc agcctacctc 240
ggtggcaagg accccaggat taaaggtggt ccctcactac aacaagtcat gagggaccag 300
ctgaagccat ttacagaacc tagaggtagg caaccaaaac caagtgtatt ggaggctgcc 360
aagaagacca tcattaatgt acttgaacaa acaatagacc cacctcaaaa gtggacattc 420
tcacaggctt gtgcctccct tgacaagacc acctctagtg gccacccaca ccacatgaga 480
aagaatgact gttggaatgg ggaatctttc acaggcaaat tggcagatca ggcctcaaag 540
gccaatttga tgtatgagga ggggaaaaat atgacaccag tttacacagg tgccctcaag 600

gatgagctgg tcaagacttg acaaaatt 628

<210> 9

<211> 452

<212> DNA

<213> Human calicivirus

<220> DNA

<223>

<400> 9

aaattttgga gatcatccac agcaccactc ccacctggta cctatgaacc agcctacctt 60

ggcggcaagg accccagagt caagggtggt ccttcattgc aacaagttat gagggaccag 120

ctgaaaccat tcactgagcc caggggcaaa ccaccaaaac caagtgtgtt agaggctgcc 180

aagaaaacca tcatcaatgt tcttgagcaa acaattgatc cacctcaaaa atggtcattc 240

gcgcaagcat gcgcatccct cgacaagacc acctctagtg gtcacccgca ccacatgcgg 300

aaaaacgact gctggaacgg ggagtccttc acaggcaaat tggcagacca ggcttccaag 360

gctaacctga tgtacgaaga gggaaagaac atgaccccag tttacacggg tgcgcttaag 420

gacgaagctg gtcaagactg acaaaatttt tt 452

<210> 10

<211> 621

<212> DNA

<213> Human calicivirus

<220> DNA

<223>

<400> 10

tgtggtcatt ggcgtgcaca ccgcagcagc acgtggagga aacacagtta tatgtgccac 60

gcaaggaagc gagggtgaag ccactcttga gggaggtgac gataagggca catattgtgg 120

agcccccatt ttgggacctg gtaatgcgcc aaagttgagc acaaaaacaa aattctggag 180

atcttccaac gcaccgctcc cgccaggcac ctatgaacca gcatacctag gtgggaaaga 240

tcaacgtgtg aagggcggtc cgtctctgca acaggtcatg agagaccaac ttaaaccttt 300

cacagagccc agaggaaaac caccaaaccc gagtgttcta gaatcagcaa agaagactat 360

tatcaatgtt ctagagcagg ttattgaccc cccccagaag tggtcctatg ctcaggcttg 420

tgcttccctt gacaaaacaa cctccagtgg acacccacac cacgttcgga agaatgatta 480

ctggagtggt gaatccttta caggaaaact tgcagaccag gcttcaaaag caaatctcat 540

gtatgaagaa ggcaaacaca tgccaccggt ttacaccgcc gcgctcaagg atgagctggt 600

gaagactgac aaaatttttt t 621

<210> 11

<211> 608

<212> DNA

<213> Human calicivirus

<220> DNA

<223>

<400> 11

gagtccacac ggctgccgct cgtgggggga acactgtcat atgtgccacc caggggagtg 60

agggggaggc tacacttgaa ggtggtgaca gtaagggaac atactgtggt gcaccaatcc 120

taggcccagg gagtgcccca aaactcagca ccaagaccaa attctggaga tcgtccacaa 180

caccactccc acctggcacc tatgaaccag cctatcttgg tggtaaggac cccagagtca 240

agggtggccc ttcattgcaa caagttatga gggatcagtt gaaaccattt acagagccca 300

ggggcaaacc accaaagcca agtgtgttgg aagctgccaa gaaaaccatc atcaatgtcc 360

ttgaacaaac aatcgatcca cccgagaaat ggtcattcac gcaagcttgc gcgtcccttg 420

acaagaccac ttccagtggc catccacacc acatgcggaa aaacgactgc tggaacgggg 480

agtccttcac aggcaagctg gcagaccagg cttccaaggc caacctgatg ttcgaagagg 540

ggaagaacat gaccccagtc tacacaggtg cgcttaagga tgagttagtt aagactgaca 600

aaattatt 608

<210> 12

<211> 603

<212> DNA

<213> Human calicivirus

<220> DNA

<223>

<400> 12

gtccacacgg ctgccgctcg tgggggaaac actgtcatat gtgccaccca ggggagtgag 60

ggggaggcta cacttgaagg tggtgacagt aagggaacat actgtggtgc accaatccta 120

ggcccaggga gtgccccaaa actcagcacc aagaccaaat tctggagatc gtccacaaca 180

ccactcccac ctggcaccta tgaaccagcc tatcttggtg gtaaggaccc cagagtcaag 240

ggtggccctt cattgcaaca agttatgagg gatcagttga aaccatttac agagcccagg 300

ggcaaaccac caaagccaag tgtgttggaa gctgccaaga aaaccatcat caatgtcctt 360

gaacaaacaa tcgatccacc cgagaaatgg tcattcacgc aagcttgcgc gtcccttgac 420

aagaccactt ccagtggcca tccacaccac atgcggaaaa acgantgctg gaacggggag 480

tccttcacag gcaagctggc agaccaggct tccaaggcca acctgatgtt cgaagagggg 540

aagaacatga ccccagtcta cacaggtgcg cttaaggatg agttagttaa gactgacaaa 600

att 603

<210> 13

<211> 606

<212> DNA

<213> Human calicivirus

<220> DNA

<223>

<400> 13

gtccacacgg ctgccgctcg tggggggaac actgtcatat gtgccaccca ggggagtgag 60

ggggaggcta cacttgaagg tggtgacagt aagggaacat actgtggtgc accaatccta 120

ggcccaggga gtgccccaaa actcagcacc aagaccaaat tctggagatc gtccacaaca 180

ccactcccac ctggcaccta tgaaccagcc tatcttggtg gtaaggaccc cagagtcaag 240

ggtggccctt cattgcaaca agttatgagg gatcagttga aaccatttac agagcccagg 300

ggcaaaccac caaagccaag tgtgttggaa gctgccaaga aaaccatcat caatgtcctt 360

gaacaaacaa tcgatccacc cgagaaatgg tcattcacgc aagcttgcgc gtcccttgac 420

aagaccactt ccagtggcca tccacaccac atgcggaaaa acgactgctg gaacggggag 480

tccttcacag gcaagctggc agaccaggct tcaaggccaa cctgatgttc gaagagggga 540

agaacatgac cccagtctac acaggtgcgc ttaaggatga gttagttaag actgacaaaa 600

ttattg 606

<210> 14

<211> 583

<212> DNA

<213> Human calicivirus

<220> DNA

<223>

<400> 14

acactgtcat atgtgccacc caggggagtg agggggaggc tacacttgaa ggtggtgaca 60

gtaagggaac atactgtggt gcaccaatcc taggcccagg gagtgcccca aaactcagca 120

ccaagaccaa attctggaga tcgtccacaa caccactccc acctggcacc tatgaaccag 180

cctatcttgg tggtaaggac cccagagtca agggtggccc ttcattgcaa caagttatga 240

gggatcagtt gaaaccattt acagagccca ggggcaaacc accaaagcca agtgtgttgg 300

aagctgccaa gaaaaccatc atcaatgtcc ttgaacaaac aatcgatcca cccgagaaat 360

ggtcattcac gcaagcttgc gcgtcccttg acaagaccac ttccagtggc catccacacc 420

acatgcggaa aaacgactgc tggaacgggg agtccttcac aggcaagctg gcagaccagg 480

cttcaaggcc aacctgatgt tcgaagaggg gaagaacatg accccagtct acacaggtgc 540

gcttaaggat gagttagtta agactgacaa aattattgga aaa 583

<210> 15

<211> 20

<212> DNA

<213> Artificial DNA

<220> DNA

<223>

<400> 15

gaggcagccg ttgaaactgt 20

<210> 16

<211> 21

<212> DNA

<213> Artificial DNA

<220> DNA

<223>

<400> 16

tccttagacg ccatcatcat t 21

<210> 17

<211> 25

<212> DNA

<213> Artificial DNA

<220> DNA

<223>

<400> 17

ctctcaacta aaacaaaatt ctgga 25

<210> 18

<211> 25

<212> DNA

<213> Artificial DNA

<220> DNA

<223>

<400> 18

catcaccata gaatgagaaa tatga 25

<210> 19

<211> 21

<212> DNA

<213> Artificial DNA

<220> DNA

<223>

<400> 19

ctgcccctac atctacaaga g 21

<210> 20

<211> 22

<212> DNA

<213> Artificial DNA

<220> DNA

<223>

<400> 20

tttgccataa ttttgtcagt ct 22